# EUROPÄISCHE PATENTSCHRIFT

(11) **EP 0 758 225 B1**
(45) Veröffentlichungstag und Bekanntmachung des Hinweises auf die Patenterteilung: **18.03.1998**
(21) Anmeldenummer: 95918575.2
(22) Anmeldetag: 20.04.1995
(51) Int. Cl.: A61K 9/00

(54) **MAGENSÄURE-BINDENDE KAUPASTILLEN**
CHEWABLE ANTI-GASTRIC ACIDITY PASTILLES
PASTILLES A MACHER ANTI-ACIDITE GASTRIQUE

(30) Priorität: 06.05.1994 DE 4415999
(43) Veröffentlichungstag der Anmeldung: 19.02.1997
(73) Patentinhaber: BOLDER ARZNEIMITTEL GmbH, D-50968 Köln (DE)
(72) Erfinder: BOLDER, Hermann-Josef, 50698 Köln (DE); IMER, Faruk, 50737 Köln (DE)
(74) Vertreter: Jönsson, Hans-Peter, Dr.Dipl.-Chem.
(86) Internationale Anmeldenummer: EP9501493
(87) Internationale Veröffentlichungsnummer: WO9530407

(56) Entgegenhaltungen:
- EP-A- 0 286 085
- EP-A- 0 338 861
- WO-A-93/10797
- WO-A-95/05165
- GB-A- 2 033 915

## Beschreibung

Die Erfindung betrifft Kau-Pastillen auf der Basis bestimmter natürlicher und/oder synthetischer Polymere, enthaltend Antacida als Wirkstoff.

Unter Pastillen - vgl. auch W. Rahn, Pharmazeutische Zeitung, S. 2214-2218 (1982) - versteht man im allgemeinen Zubereitungen, die im Munde gelutscht oder zerkaut werden. Und zwar unterscheidet man im wesentlichen zwischen Tabletten, Hartbonbons und Gummipastillen (auch genannt Gummibonbons).

Die Verfahren zur Herstellung dieser Darreichungsformen unterscheiden sich grundsätzlich voneinander.

Tabletten werden auf Tablettenmaschinen gepreßt. Die Tablettenmasse muß dazu durch Mischen und Granulieren vorbereitet werden. Verschiedene Autoren haben sich bereits mit Granulierungsmethoden schwierig zu verarbeitender Wirkstoffe beschäftigt.

Bonbons werden in der Weise hergestellt, daß man Saccharose und Glucosesirup mischt, bei ca. 130°C kocht und der Masse im Vakuum das Wasser bis auf 0,5 bis 2 % entzieht. Der auf ca. 85°C abgekühlten zähen Bonbonmasse werden die Wirk- und Aromastoffe zugesetzt und diese durch Kneten untergemischt. In Kegelrollern und anderen Bonbonmaschinen wird die Bonbonmasse unter stetigem Abkühlen zu Strängen ausgezogen, geformt und geschnitten. Bekanntermaßen ist die Wirkstoffverteilung, bedingt durch die Zähigkeit der Bonbonmasse, ziemlich ungenau.

Gummipastillen werden in der Weise hergestellt, daß man zunächst in einem Rührwerkskessel Hydrokolloide, wie z. B. Gummi arabicum, zusammen mit Saccharose, Glucosesirup, Sorbitol, Xylitol o.ä. in Wasser auflöst und in dieser Grundmasse die Wirkstoffe gelöst, emulgiert bzw. suspendiert. Die so erhaltene Gießmasse wird in sogenannten Puderhorden ausgegossen. Dies sind beispielsweise flache Holzkisten von ca. 80 x 40 cm, die mit Stärke, insbesondere Maisstärke, gefüllt sind. In dem glattgestrichenen Puder werden mittels eines Stempelbretts die gewünschten Formen eingedrückt und in die erhaltenen Vertiefungen die warme Gießmasse genau dosiert eingepumpt, wobei sich die eingegossene Masse nicht mit dem Puder verbindet. Horde um Horde, je 500 bis 1000 Pastillen, wird so gegossen, gestapelt und in Trockenkammern im Verlauf von 3 bis 4 Tagen den Pastillen das Wasser bis auf etwa 10 % Restfeuchte entzogen. Die so gefertigten Pastillen werden "ausgepudert" und dann einer Endbehandlung zugeführt.

Gegenstand der DE 41 40 116 A1 sind Pastillen auf der Basis natürlicher und/oder synthetischer Polymere oder fettartiger Substanzen mit Zucker und/oder Zuckeraustauschstoffen, enthaltend Poly-(dimethylsiloxane) (Dimeticon, Simeticone) sowie ein Verfahren zu ihrer Herstellung.

Nach Römpp Chemie Lexikon 9. Auflage, Seit 200, 1989 versteht man unter dem Begriff "Antacida" Stoffe, die einer Hyperacidität des Magensafts entgegen wirken sollen. Geeignete Verbindungen sind unter anderem Magnesiumhydroxid, Magnesiumoxid, Magnesiumcarbonat, Magnesiumsilikat, Alumuminiumhydroxide, Aluminiumphosphat, Magnesium-Aluminium-Silikate, Hydrotalcid und Magaldrat. Nach Römpp sollte zwar von der Verwendung des früheren Natriumhydrogencarbonats und Calciumcarbonats abgesehen werden, jedoch sind zahlreiche Handelsprodukte erhältlich, die diese Bestandteile wenigstens in untergeordneten Anteilen enthalten.

Die Aufgabe der vorliegenden Erfindung bestand gegenüber dem Stand der Technik darin, unter Anwendung eines an sich aus der Süßwarenherstellung bekannten Gieß-Verfahrens, eine neue einzeldosierbare Darreichungsform für Antacida zur Verfügung zu stellen, die sich auszeichnet durch: feinste Verteilung des Wirkstoffes in der Pastille, sehr genaue Einzeldosierung des Wirkstoffes, einfache Handhabung des Wirkstoffes, vor allem angenehme Einnahme des Arzneimittels und optimale zeitliche Streckung des Wirkstoffes im Magen durch das langsame Kauen der Pastillen.

Gelöst wird die vorgenannte Aufgabe durch Pastillen auf der Basis von wenigstens teilweise oder vollständig wasserlöslichen, natürlichen und/oder synthetischen Polymeren, ausgewählt aus Gummen, Alginaten, Carragen, Stärke und Pektin, die in wäßrigen Systemen Gele oder viskose Lösungen bilden und weiteren Hilfs- und Zusatzstoffen, enthaltend Antacida als Wirkstoff.

Es wurde gefunden, daß die teils wasserlöslichen, teils wasserunlöslichen Antacida sich in außerordentlich einfacher Weise feindispers in Pastillen auf der Basis der genannten natürlichen und/oder synthetischen Polymere einarbeiten lassen.

Der Begriff Pastillen und insbesondere der Begriff Gummipastillen beinhaltet im Sinne der vorliegenden Erfindung solche, die durch Gießen hergestellt werden. Dementsprechend bestehen die Pastillen gemäß der vorliegenden Erfindung aus verschiedenen geformten elastischen Formkörpern, die in einer Mischung von Hydrokolloiden und weiteren Hilfs- und Zusatzstoffen Antacida in feinster Verteilung enthalten. Gummipastillen werden als feste Lösungen bezeichnet, die beim Lutschen wieder in flüssige Lösungen oder Dispersionen zurückverwandelt werden. Mit Hilfe der vorliegenden Erfindung ist es möglich, eine genaue Einzeldosierung der Pastille bei relativ schonender Verarbeitungsweise der Bestandteile zu erreichen. Bei den genannten, wenigstens teilweise oder vollständig wasserlöslichen, natürlichen und/oder synthetischen Polymeren, ist eine besonders gute Einarbeitung der Antacida möglich, da hier bei relativ niedrigen Temperaturen gearbeitet werden kann. Somit entsteht eine besonders homogene Verteilung des Wirkstoffes in der Gesamtmasse, die es erlaubt, die Dosierung des Wirkstoffes mit Standardabweichungen im Bereich von 0,5 bis 2 % zu dosieren. Darüber hinaus ist mit Hilfe des erfindungsgemäßen Verfahrens die Herstellung von relativ hochkonzentrierten Antacida-Pastillen möglich.

Besonders bevorzugte natürliche und/oder synthetische Polymere im Sinne der vorliegenden Erfindung sind auch unter dem Begriff "Hydrokolloide" bekannt. Besonders bevorzugt sind die Gummen, ausgewählt aus Gummi arabicum oder Traganth. In gleicher Weise sind die Hilfs- und Zusatzstoffe vorzugweise ausgewählt aus Zucker und/oder Zuckeraustauschstoffen, hydrierten Fetten, Stearinsäure, Paraffinen, Oligosacchariden, Polysacchariden und/oder Dextran.

Entsprechende Pastillen mit anderen Wirkstoffen sind an sich im Stand der Technik unter dem Begriff "Gummipastillen" bekannt. Diese leiten ihren Namen von dem in ihnen verarbeiteten Rohstoff Gummi arabicum ab. Auch im Sinne der vorliegenden Erfindung wird dieses Hydrokolloid bevorzugt als Grundstoff verwendet, weil es der Pastille eine gute Lutsch- und Kaueigenschaft verleiht.

Neben den Polymeren enthält die Grundmasse insbesondere Geschmacksträger wie Zucker und/oder Zuckeraustauschstoffe, da der Patient die Pastille kauen soll. Dementsprechend ist es erforderlich, daß die Pastillen so gut schmecken, daß diese nicht verweigert oder aber hinuntergeschluckt werden. Zur Geschmacksverbesserung werden, wie an sich im Stand der Technik bekannt, entsprechende Hilfsstoffe wie Saccharose oder deren Austauschstoffe Fructose, hydrierter Glucosesirup, Sorbit, Mannit und/oder Xylit sowie bekannte Süßstoffe eingesetzt. Daneben können auch Geschmackskorrigenzien und Essenzen, ebenso wie etherische Öle, eingesetzt werden.

Die Mengenanteile der jeweils notwendigen Bestandteile der Pastillen sind weniger kritisch. Dementsprechend enthält die Grundmasse beispielsweise 2 bis 80 Gew.-% der Polymere, bezogen auf die Gesamtmasse der Pastillen. Besonders bevorzugt ist eine Menge von 10 bis 60 Gew.-% Gummi arabicum, bezogen auf die Gesamtmasse der Pastillen.

In einer weiteren bevorzugten Ausführungsform der vorliegenden Erfindung enthalten Pastillen 20 bis 50 Gew.-% Zucker und/oder Zuckeraustauschstoffe, bezogen auf die Gesamtmasse der Pastillen. Die Menge an Antacida kann bei den erfindungsgemäßen Pastillen in einem breiten Bereich variiert werden. Vorzugsweise sollte die Menge an Antacida bezogen auf die Gesamtrezeptur so groß wie möglich sein, mindestens aber ein Säurebindungsvermögen von 20 - 25 mval je Einzeldosis aufweisen.

Im Handel erhältliche Präparate, wie Suspensionen oder Kautabletten mit säurebindenden Wirkstoffen enthalten Antacida mit einem Säurebindungsvermögen von 10 - 25 mval. Dementsprechend besteht eine besonders bevorzugte Ausführungsform der vorliegenden Erfindung darin, Pastillen mit einer Säurebindungskapazität von 10 - 40 mval zur Verfügung zu stellen.

Bei der Herstellung der Pastillen werden insbesondere die oben definierten Polymere mit Wasser und weiteren Hilfs- und Zusatzstoffen unter Bildung eine Gels oder einer viskosen Lösung in Kontakt gebracht. Anschließend werden Antacida in der so erhaltenen Grundmasse suspendiert oder gelöst und dann diese flüssige Masse in Formen gegossen, bei Raumtemperatur oder bei erhöhter Temperatur, insbesondere 40 bis 70°C, bevorzugt 40 bis 50°C, getrocknet, aus der Form entfernt und einer Endbehandlung zugeführt.

Beispielsweise werden zu Beginn des Herstellungsverfahrens Gelatine und Gummi arabicum in Wasser gelöst und mit Antacida suspendiert. Diese Arzneimittelmischung wird in sogenannten Puderhorden ausgegossen und, wie eingangs beschrieben, getrocknet, vom Puder getrennt und endbehandelt. Die besonderen Vorteile der erfindungsgemäßen Pastillen und des Verfahrens zu ihrer Herstellung bestehen in einer geringen Temperaturbelastung der Hilfs- und Wirkstoffe, deren vollständiger Homogenität in der Gießmasse, die eine hohe Genauigkeit der Wirkstoffdosierung erlaubt, vor allem aber die hervorragende Kaufähigkeit gegenüber Tabletten und der bessere Geschmack, alles Vorzüge, die die Compliance des Patienten fördern.

## Patentansprüche

1. Kaupastillen auf der Basis von wenigstens teilweise oder vollständig wasserlöslichen natürlichen und/oder synthetischen Polymeren, ausgewählt aus Gummen, Alginaten, Carragen, Stärke und Pektin, die in wäßrigen Systemen Gele oder viskose Lösungen bilden und weiteren Hilfs- und Zusatzstoffen, enthaltend Antacida als Wirkstoff.

2. Pastillen nach Anspruch 1, dadurch gekennzeichnet, daß die Gummen ausgewählt sind aus Gummi arabicum oder Traganth.

3. Pastillen nach Anspruch 1 oder 2, dadurch gekennzeichnet, daß die Hilfs- und Zusatzstoffe ausgewählt sind aus Zucker und/oder Zuckeraustauschstoffen, hydrierten Fetten, Stearinsäure, Paraffinen, Oligosacchariden, Polysacchariden und/oder Dextran.

4. Pastillen nach einem oder mehreren der Ansprüche 1 bis 3, enthaltend als Zuckeraustauschstoff Fructose, Sorbit, Mannit, Xylit, hydrierten Glucosesirup und/oder Süßstoffe.

5. Pastillen nach einem oder mehreren der Ansprüche 1 bis 4, enthaltend 2 bis 80 Gew.-% Polymere, bezogen auf die Gesamtmasse der Pastillen.

6. Pastillen nach einem oder mehreren der Ansprüche 1 bis 5, enthaltend 10 bis 60 Gew.-% Gummi arabicum, bezogen auf die Gesamtmasse der Pastillen.

7. Pastillen nach einem oder mehreren der Ansprüche 1 bis 6, enthaltend 20 bis 50 Gew.-% Zucker und/oder Zuckeraustauschstoffe, bezogen auf die Gesamtmasse der Pastillen.

8. Pastillen nach einem oder mehreren der Ansprüche 1 bis 7, mit einer Säurebindungskapazität von 10 - 40 mval, insbesondere 10 bis 25 mval je Einzeldosis.

9. Pastillen nach einem oder mehreren der Ansprüche 1 bis 8, dadurch gekennzeichnet, daß die Antacida ausgewählt sind aus Aluminiumhydroxiden, Aluminiumphosphaten, Magnesiumhydroxid, Magnesiumoxid, Magnesiumcarbonat, Magnesiumsilikat, Magnesium-Aluminium-Silikaten, Hydrotalcid und Magaldrat, Natriumcarbonat und Calciumcarbonat und deren Gemischen.

10. Verfahren zur Herstellung von Kaupastillen nach einem oder mehreren der Ansprüche 1 bis 9, wobei man
a) wenigstens teilweise oder vollständig wasserlösliche natürliche und/oder synthetische Polymere, ausgewählt aus Gummen, Alginaten, Carragen, Stärke und Pektin, mit Wasser und weiteren Hilfs- und Zusatzstoffen unter Bildung eines Gels oder einer viskosen Lösung in Kontakt bringt und Antacida in der so enthaltenden Grundmasse emulgiert, suspendiert und löst,
b) die Emulsion, Suspension oder Lösung gemäß a) in Formen ausgießt, die gegebenenfalls mittels Stempelbrettern in Puderhorden eingedrückt sind und
c) bei Raumtemperatur oder erhöhter Temperatur trocknet.

11. Verfahren nach Anspruch 10, dadurch gekennzeichnet, daß man die Pastillen bei einer Temperatur im Bereich von 40 bis 70°C, insbesondere 40 bis 50°C, trocknet.

12. Verfahren nach Anspruch 10 oder 11, dadurch gekennzeichnet, daß man die getrockneten Pastillen aus der Form entfernt und gegebenenfalls endbehandelt.

## Claims

1. Chewing pastilles based on at least partially or completely water soluble natural and/or synthetic polymers, selected from gums, alginates, carrageen, starch, and pectin, which will form gels or viscous solutions in aqueous systems, and additional adjuvants and additives, said pastilles containing antacids as active ingredients.

2. The pastilles according to claim 1, characterized in that said gums are selected from gum arabic or tragacanth.

3. The pastilles according to claim 1 or 2, characterized in that said adjuvants and additives are selected from sugar and/or sugar substitutes, hydrogenated fats, stearic acid, paraffins, oligosaccharides, polysaccharides, and/or dextran.

4. The pastilles according to one or more of claims 1 to 3, containing fructose, sorbitol, mannitol, xylitol, hydrogenated glucose syrup, and/or artificial sweeteners as said sugar substitutes.

5. The pastilles according to one or more of claims 1 to 4, containing from 2 to 80% by weight of polymers, based on the total mass of the pastilles.

6. The pastilles according to one or more of claims 1 to 5, containing from 10 to 60% by weight of gum arabic, based on the total mass of the pastilles.

7. The pastilles according to one or more of claims 1 to 6, containing from 20 to 50% by weight of sugar and/or sugar substitutes, based on the total mass of the pastilles.

8. The pastilles according to one or more of claims 1 to 7, having an acid binding capacity of from 10 to 40 mval, in particular from 10 to 25 mval, per unit dose.

9. The pastilles according to one or more of claims 1 to 8, characterized in that said antacids are selected from aluminum hydroxides, aluminum phosphates, magnesium hydroxide, magnesium oxide, magnesium carbonate, magnesium silicate, magnesium aluminum silicates, hydrotalcite and magaldrate, sodium carbonate and calcium carbonate, and mixtures thereof.

10. A process for the preparation of the chewing pastilles according to one or more of claims 1 to 9, wherein
a) at least partially or completely water soluble natural and/or synthetic polymers, selected from gums, alginates, carrageen, starch, and pectin, are contacted with water and additional adjuvants and additives to form a gel or a viscous solution, and antacids are emulsified, suspended or dissolved in the base composition thus obtained;
b) the emulsion, suspension or solution according to a) is cast into molds which are optionally stamped into powder trays by means of stamping boards; and
c) dried at room temperature or elevated temperature.

11. The process according to claim 10, characterized in that said pastilles are dried at a temperature in the range of from 40 to 70°C, particularly from 40 to 50°C.

12. The process according to claim 10 or 11, characterized in that the dried pastilles are removed from the mold and optionally subjected to final treatment.

## Revendications

1. Pastilles à mâcher à base de polymères naturels et/ou de synthèse au moins partiellement ou totalement solubles dans l'eau choisis entre les gommes, les alginates, le carragheen, l'amidon et la pectine qui, dans des systèmes aqueux, forment des gels ou des solutions visqueuses, et à base d'autres adjuvants et additifs, contenant des antiacides comme principe actif.

2. Pastilles selon la revendication 1, caractérisées en ce que les gommes sont choisies dans le groupe comprenant la gomme arabique et la gomme adragante.

3. Pastilles selon la revendication 1 ou la revendication 2, caractérisées en ce que les adjuvants et additifs sont choisis dans le groupe comprenant le sucre et les substituts du sucre, les matières grasses hydrogénées, l'acide stéarique, les paraffines, les oligosaccharides, les polysaccharides et/ou le dextrane.

4. Pastilles selon l'une ou plusieurs des revendications 1 à 3, contenant comme substitut du sucre du fructose, du sorbitol, du mannitol, du xylitol, du sirop de glucose hydrogéné et/ou des édulcorants.

5. Pastilles selon l'une ou plusieurs des revendications 1 à 4, contenant de 2 à 80 % en poids de polymères, ramené à la masse totale des pastilles.

6. Pastilles selon l'une ou plusieurs des revendications 1 à 5, contenant de 10 à 60 % en poids de gomme arabique, ramené à la masse totale des pastilles.

7. Pastilles selon l'une ou plusieurs des revendications 1 à 6, contenant de 20 à 50 % en poids de sucre ou de substituts du sucre, ramené à la masse totale des pastilles.

8. Pastilles selon l'une ou plusieurs des revendications 1 à 7, présentant une capacité de liaison des acides de 10 à 40 mval, et notamment de 10 à 25 mval, par dose unitaire.

9. Pastilles selon l'une ou plusieurs des revendications 1 à 8, caractérisées en ce que les antiacides sont choisis dans le groupe comprenant les hydroxydes d'aluminium, les phosphates d'aluminium, l'hydroxyde de magnésium, l'oxyde de magnésium, le carbonate de magnésium, le silicate de magnésium, les silicates de magnésiumaluminium, l'hydrotalcide et le magaldrate, le carbonate de sodium et le carbonate de calcium, et leurs mélanges.

10. Procédé de fabrication de pastilles à mâcher selon l'une ou plusieurs des revendications 1 à 9, consistant à
a) mettre en contact des polymères naturels et/ou de synthèse au moins partiellement ou totalement solubles dans l'eau choisis entre les gommes, les alginates, le carragheen, l'amidon et la pectine avec de l'eau et d'autres adjuvants et additifs afin de former un gel ou une solution visqueuse, et à émulsionner, mettre en suspension et dissoudre des antiacides dans la masse primaire ainsi obtenue ;
b) verser l'émulsion, la suspension ou la solution obtenue à l'étape a) dans des moules qui sont le cas échéant poinçonnés au moyen de planches de poinçonnage dans des claies de poudre ; et
c) sécher à la température ambiante ou à une température plus élevée.

11. Procédé selon la revendication 10, caractérisé en ce que le séchage des pastilles se fait à une température de 40 à 70 °C, et notamment de 40 à 50 °C.

12. Procédé selon la revendication 10 ou la revendication 11, caractérisé en ce que les pastilles séchées sont démoulées et le cas échéant soumises à un traitement de finition.
